# EUROPÄISCHE PATENTSCHRIFT

(11) **EP 2 291 140 B1**
(45) Veröffentlichungstag und Bekanntmachung des Hinweises auf die Patenterteilung: **07.03.2018**
(21) Anmeldenummer: 09768687.7
(22) Anmeldetag: 19.06.2009
(51) Int. Cl.: A61C 8/00

(54) **IMPLANTATIONSVORRICHTUNG, IMPLANTAT UND WERKZEUG**
IMPLANTATION DEVICE, IMPLANT AND TOOL
DISPOSITIF D'IMPLANTATION, IMPLANT ET OUTIL

(30) Priorität: 23.06.2008 CH 9592008
(43) Veröffentlichungstag der Anmeldung: 09.03.2011
(73) Patentinhaber: New Dent AG, 4702 Oensingen (CH)
(72) Erfinder: PERLER, Peter, 2562 Port (CH)
(74) Vertreter: Frei Patent Attorneys
(86) Internationale Anmeldenummer: PCT/CH2009/000211
(87) Internationale Veröffentlichungsnummer: WO 2009/155718

(56) Entgegenhaltungen:
- WO-A1-00/12031
- WO-A2-2006/011127
- US-A- 5 791 899
- US-A1- 2006 100 697
- US-A1- 2007 191 879

## Beschreibung

Die Erfindung liegt im Bereich der Dentalmedizin und betrifft eine Implantationsvorrichtung, ein Implantat und ein Werkzeug oder ein Zwischenstück nach den Oberbegriffen der entsprechenden unabhängigen Patentansprüche. Die erfindungsgemässe Implantationsvorrichtung weist ein Implantationswerkzeug oder ein Zwischenstück auf sowie ein Implantat, wobei das Implantat an das Werkzeug oder Zwischenstück gekoppelt oder koppelbar ist. Das Implantationsverfahren, das in diesem Patentschrift beschrieben wird und das nicht Teil der Erfindung ist, dient zur Implantation des Implantates unter Verwendung eines Werkzeuges mit oder ohne Zwischenstück, wobei für die Implantation das Implantat an das Werkzeug oder Zwischenstück gekoppelt ist und nach der Implantation das Werkzeug oder Zwischenstück vom Implantat entkoppelt wird.

In der vorliegenden Patentschrift wird der Begriff Implantat verwendet für ein in einem menschlichen oder tierischen Körper oder gegebenenfalls an einem menschlichen oder tierischen Körper zu positionierendes und gegebenenfalls zu befestigendes Element. Das in der Patentschrift vorwiegend beschriebene Implantat ist ein Dentalimplantat. Unter dem Begriff Implantat nach der obigen Definition sind aber beispielsweise auch zu verstehen: Wurzelstifte, Abutments, Zahnkronen, Zahnfüllungen. In der vorliegenden Patentschrift wird der Begriff Implantationsverfahren verwendet für ein Verfahren zur Positionierung und gegebenenfalls Befestigung des Implantates in oder an einem menschlichen oder tierischen Körper, wobei für die Positionierung und/oder Befestigung auf das Implantat gegebenenfalls Kräfte auszuüben sind oder Energie beispielsweise in Form von Wärme in das Implantat einzukoppeln ist. Das in der Patentschrift vorwiegend beschriebene Implantationsverfahren ist eine Implantation mittels mechanischer Vibration (insbesondere Ultraschall-Vibration) und eines Materials, das durch die mechanische Vibration verflüssigbar ist. Unter dem Begriff Implantationsverfahren nach der obigen Definition sind aber beispielsweise auch zu verstehen: Einschrauben von schraubenförmigen Implantaten, Befestigen von Implantaten mittels Zement oder Klebstoff, Einbringen von Implantaten in Gewebeöffnungen zur Erzeugung eines Press-fits, Einschlagen von mit Spitzen oder Schneidekanten ausgerüsteten Implantaten in Gewebe etc.

In der vorliegenden Patentschrift wird der Begriff Implantationswerkzeug verwendet für ein Werkzeug, das verwendet wird für die Implantation des Implantates, das heisst, insbesondere für das Positionieren und gegebenenfalls auch für das Befestigen des Implantats im oder am menschlichen oder tierischen Körper. Das Werkzeug wird üblicherweise von Hand geführt und kann für die Implantation an einen Antrieb angeschlossen sein (z.B. an Schwingungsantrieb anschliessbare Sonotrode oder an einen Rotationsantrieb anschliessbares Schraubwerkzeug). Wenn kein Zwischenstück (siehe unten) verwendet wird, wird das Implantat für die Implantation an einen distalen Werkzeugbereich angekoppelt und das Werkzeug wird nach der Implantation vom Implantat entkoppelt.

In der vorliegenden Patentschrift wird der Begriff Zwischenstück verwendet, für ein zwischen Implantat und Implantationswerkzeug angeordnetes oder anzuordnendes Element im Sinne eines Adapters. Das Zwischenstück wird für die Implantation in an sich bekannter Art und Weise an einem Werkzeug befestigt oder kooperiert mit diesem und das Implantat ist in einem Implantationsverfahren mit Verwendung eines Zwischenstückes in der oben für das Implantationswerkzeug beschriebenen Art und Weise an einem distalen Bereich des Zwischenstücks angekoppelt.

Das weiter oben bereits angetönte Implantationsverfahren, in dem ein durch mechanische Vibration (z.B. Ultraschallschwingungen) verflüssigbares Material und mechanische Vibrationen (z.B. Ultraschall) verwendet werden, ist bekannt beispielsweise aus den Publikationen US 7,335,205, US 6,955,540, US 7,008,226 und WO2005/07969, die eine Implantationsvorrichtung gemäss dem Oberbegriff des Anspruchs 1 offenbart. In diesen Verfahren wird das Implantat, das ganz oder mindestens in Oberflächenbereichen aus thermoplastischem Material besteht, in Schwingungen versetzt und gleichzeitig gegen die Oberfläche (z.B. Knochengewebe oder Dentin) gepresst, an der es befestigt werden soll (z.B. innere Oberfläche einer Gewebeöffnung). Dazu wird beispielsweise eine an einen Ultraschallgenerator angeschlossene Sonotrode (Implantationswerkzeug) auf das Implantat aufgesetzt. In den Kontaktbereichen zwischen Gewebe und Implantat entstehen im schwingenden thermoplastischen Material hohe Spannungskonzentrationen, durch die das Material verflüssigt wird und in Poren, Öffnungen oder entsprechend dafür erstellte Kavitäten fliesst, um nach dem Widererstarren mit dem Gewebe eine vorteilhafterweise formschlüssige Verbindung zu bilden.

In den Publikationen US Patent 7,335,205 und US Anmeldung 2006/0 105 295 sind auch miteinander kooperierende Strukturen von proximalen Implantatbereichen und distalen Sonotroden- oder Adapter-Bereichen beschrieben, die sich für eine Ankoppelung des Implantates an die Sonotrode (Implantationswerkzeug) oder den Adapter (Zwischenstück) eignen, derart, dass das Implantat mit Hilfe des Werkzeuges positioniert und mindestens ein Teil der Ultraschallschwingungen von der Sonotrode oder dem Adapter auf das Implantat übertragen werden können. Die beschriebenen Kopplungen sind beispielsweise elastische Schnappverbindungen, Bolzenverbindungen, Schraubverbindungen oder Verbindungen mittels Kapillarkräften. Die genannten Verbindungen sind möglichst starr und vorteilhafterweise vorgespannt, um die Schwingungen vollständig und möglichst verlustfrei auf das Implantat übertragen zu können, sie können aber auch Spielpassungen aufweisen, derart, dass nur ein gegen das Implantat wirkender Teil der Schwingungen (Druck), nicht aber ein vom Implantat weg gerichteter Teil der Schwingungen (Zug) auf das Implantat übertragen wird und damit die Sonotrode oder der Adapter hämmernd auf das Implantat wirkt.

Die beschriebenen Kopplungen sind zum Teil an Implantationsorten mit engen Platzverhältnissen nach der Implantation nicht einfach entkoppelbar und insbesondere die starren und vorgespannten Kopplungen können nur entkoppelt werden mittels Kräften (z.B. Zug oder Drehmoment), die vom frisch implantierten Implantat aufgenommen werden müssen, was in vielen Anwendungen nicht optimal ist.

Es ist die Aufgabe der vorliegenden Erfindung eine Implantationsvorrichtung zu schaffen, mit denen es möglich wird, ein für die Implantation verwendetes Implantationswerkzeug oder Zwischenstück, an das das Implantat für die Implantation (Positionierung und gegebenenfalls Befestigung) gekoppelt ist, nach der Implantation vom Implantat zu entkoppeln, ohne dass bei dieser Entkoppelung Kräfte auf das Implantat wirken und ohne dass für die Entkopplung ein weiteres Werkzeug notwendig ist. Vorrichtung gemäss Erfindung sollen sich insbesondere eignen für starre und vorgespannte Verbindungen zwischen dem Werkzeug oder Zwischenstück und dem Implantat, was für die Übertragung von Kräften oder Energie vom Werkzeug oder Zwischenstück auf das Implantat notwendig oder vorteilhaft sein kann. Sie sind aber selbstverständlich auch anwendbar für Verbindungen mit Spiel, die sich für die Übertragung von Kräften oder Energie nur beschränkt eignen, aber für verschiedenste Anwendungen genügend oder vorteilhaft sein können. Vorrichtung gemäss Erfindung eignen sich insbesondere auch für eine Entkopplung in wesentlich limitierten Platzverhältnissen.

Diese Aufgabe wird gelöst durch die Implantationsvorrichtung, das Implantat und das Werkzeug oder das Zwischenstück, wie sie in den unabhängigen Ansprüchen definiert sind. Die erfindungsgemässe Implantationsvorrichtung weist ein Implantationswerkzeug oder ein Zwischenstück auf sowie ein Implantat, wobei ein distaler Bereich des Werkzeugs oder Zwischenstücks und das Implantat mit kooperierenden Verbindungsstrukturen ausgerüstet sind, mit deren Hilfe das Implantat an den distalen Bereich von Werkzeug oder Zwischenstück angekoppelt oder ankoppelbar ist. Von dem kooperierenden Paar von Verbindungsstrukturen weist mindestens die eine ein Element aus einem Werkstoff mit Formgedächtnis (Formgedächtnis-Element) auf.

Der Werkstoff mit Formgedächtnis ist derart ausgewählt und das Formgedächtnis-Element derart ausgebildet, dass die Verbindungsstrukturen bei Umgebungstemperaturen und/oder Körpertemperaturen in einer verbindenden Konfiguration sind und dass die Verbindungsstrukturen in eine nicht-verbindende Kongfiguration bringbar sind durch eine Temperaturänderung von Umgebungs- oder Körpertemperatur zu einer physiologisch mindestes für kurze Zeit tolerierbaren und entweder tiefer oder höher als Umgebungs- oder Körpertemperatur liegenden Temperatur. Beispielsweise sind die Verbindungsstrukturen in der verbindenden Konfiguration bei Temperaturen tiefer als ca. 32°C und erzeugt eine Temperaturerhöhung auf 35 bis 50°C (vorteilhafterweise 40 bis 45°C) eine Formänderung des Formgedächtins-Elements und dadurch einen Übergang in die nicht-verbindende Konfiguration. Andererseits können die Verbindungsstrukturen auch in der verbindenden Konfiguration sein bei Temperaturen höher als ca. 10°C und die Formänderung und damit der Übergang in die nicht-verbindende Konfiguration durch eine Temperatursenkung auf zwischen 5 und -10°C (vorteilhafterweise zwischen 5 und 0°C) bewirkt werden.

In der erfindungsgemässen Implantationsvorrichtung anwendbare Verbindungsstrukturen für eine starre und gegebenenfalls vorgespannte Verbindung sind vorteilhafterweise für einen Form-, Mikroform- und/oder Kraftschluss ausgestaltet und weisen für die Vorspannung mindestens ein elastisch deformierbares Element auf, wobei das Formgedächnis-Element auch die Funktion des elastisch deformierbaren Elements übernehmen kann oder für die beiden Funktionen getrennte Elemente vorgesehen sind. Die beiden kooperierenden Verbindungsstrukturen sind beispielsweise ausgebildet für ein Ineinandergreifen und/oder Klemmen in der verbindenden Konfiguration und für eine Aufhebung des Ineinandergreifens und/oder der Klemmkraft beim Übergang in die nicht-verbindende Konfiguration. Verbindungen mit Spiel sind beispielsweise mit ineinandergreifenden Verbindungsstrukturen ohne elastisch deformierbares Element realisierbar.

Das Formgedächtnis-Element der erfindungsgemässen Implantationsvorrichtung besteht beispielsweise aus einer Formgedächtnis-Legierung wie beispielsweise Nitinol (Handelsname von Nickel-Titan-Legierung mit Formgedächtnis) könnte aber auch ein Formgedächtnis-Polymer aufweisen.

Die Formänderung von Formgedächtnis Legierungen basiert auf einer Strukturänderung von einer martensitischen Niedertemperaturstruktur zu einer austenitischen Hochtemperaturstruktur und/oder umgekehrt. Falls beide Strukturänderungen mittels Temperaturänderung bewirkbar sind, spricht man von einem intrinsischen Zweiwegeffekt. Bei einem extrinsischen Zweiwegeffekt wird eine der Strukturänderungen mechanisch oder mittels einer Spannung induziert. Es gibt auch Formgedächtnis-Werkstoffe mit Einwegeffekt, welche nur einmal durch Temperaturerhöhung aus einer pseudoplastischen Verformung im martensitischen Zustand bei Erwärmung in eine "erinnerte" Ursprungsform übergehen, aber beim Abkühlen nicht mehr in die gleiche pseudoplastisch verformte Martensitform zurückkehren. Für das erfindungsgemässe Verbindungssystem können sowohl Einweg- oder Zweiweg- Formgedächtnis-Effekte als auch Superelastizität der entsprechenden Materialien ausgenutzt werden.

Die Formumwandlung von Formgedächtnis-Elementen auf der Basis von Formgedächtnis-Polymeren basiert auf dem Übergang von einer harten zu einer weichen Phase bei der Glasübergangstemperatur.

Für die Formumwandlung muss das Formgedächtnis-Element erwärmt oder abgekühlt werden. Für Erwärmung und Abkühlung eignet sich im chirurgischen Umfeld insbesondere eine Wärme- oder Kälteübertragung durch eine Flüssigkeit der gewünschten Temperatur, insbesondere Wasser oder physiologische Salzlösung. Das Formgedächnis-Element oder mit dem Formgedächtins-Element in Kontakt stehende Bereiche von Werkzeug, Zwischenstück und/oder Implantat können aber auch für eine Absorption von elektromagnetischer Strahlung (insbesondere Licht) ausgerüstet sein und für eine Erwärmung bestrahlt werden. Erwärmung kann ferner erzielt werden beispielsweise durch induktive Erwärmung oder mittels elektrischem Strom, der durch einen entsprechen angeordneten Widerstand fliesst.

Das beschriebene Verfahren weist im wesentlichen die folgenden drei Schritte auf: 1. Implantieren des Implantates, wobei das Implantat an das Werkzeug gekoppelt ist oder an das Zwischenstück, das seinerseits an das Werkzeug gekoppelt ist oder mit diesem kooperiert. 2. Entkoppelung des Werkzeugs oder Zwischenstücks vom Implantat durch Erwärmen oder Abkühlen des Formgedächnis-Elements derart, dass die Verbindungsstrukturen von ihrer verbindenden Konfiguration in die nichtverbindende Konfiguration überführt werden, und 3. Entfernung des Werkzeuges oder Zwischenstücks vom Implantat, wobei bei der Durchführung der Schritte 2 und 3 keine Kräfte auf das Implantat übertragen werden und deshalb weder die Position des Implantates verändert noch eine gegebenenfalls erstellte Befestigung des Implantats am Gewebe negativ beeinflusst wird.

Vorteilhafterweise wird das Implantat herstellerseitig an das Werkzeug oder Zwischenstück gekoppelt, so dass die die Implantation ausführende Person (z.B. Chirurg) oder eine Hilfsperson gegebenenfalls das Zwischenstück noch an ein entsprechendes Werkzeug und/oder das Werkzeug an einen Antrieb anschliessen muss und dann unmittelbar zur Implantation übergehen kann. In einem solchen Falle kann das Werkzeug und insbesondere ein Zwischenstück noch andere Funktionen (z.B. Akustische Kompensation bei Implantation mit Ultraschall, Verpackung und Lagerung) übernehmen und ist das Werkzeug oder Zwischenstück vorteilhafterweise ein Einwegartikel, der nach der Implantation entsorgt wird. Gegebenenfalls wird aber auch die Kopplung des Implantats an das Werkzeug oder Zwischenstück im Operationssaal durchgeführt und gehört damit als vorbereitender Schritt zum Implantationsverfahren.

Die Kopplung des Implantats an das Werkzeug oder Zwischenstück ist beispielsweise eine einfache Umkehr des Entkopplungsschrittes (intrinsischer Zweiwegeffekt), wobei das Implantat vor der Formumwandlung des Formgedächtnis-Elements an Werkzeug oder Zwischenstück positioniert und durch die Formumwandlung daran gekoppelt wird. Das Implantat kann aber auch nach der Formumwandlung an das Werkzeug oder Zwischenstück gekoppelt werden, was eine mindestens teilweise elastische Verformbarkeit der Verbindungsstrukturen erfordert. Bei Verwendung eines Werkstoffes mit Formgedächtnis, der auf einem extrinsischen Zweiwegeffekt oder auf einem Einwegeffekt basiert, wird die verbindende Konfiguration der Verbindungsstrukturen durch mechanische Formveränderung bewirkt, die wiederum mit oder ohne für die Kopplung positioniertem Implantat und bei einer Temperatur, in der die verbindende Konfiguration stabil ist, erstellt wird.

In einem ersten Aspekt der Erfindung ist das Formgedächtnis-Element Teil der Verbindungsstruktur, die in einem distalen Bereich eines Zwischenstücks angeordnet ist. Ein proximaler Bereich des Zwischenstücks ist in an sich bekannter Art und Weise für eine Kopplung an ein Werkzeug oder für eine Kooperation mit einem Werkzeug ausgerüstet (z.B. Verschraubung, Bajonettverschluss, Schnappverbindung, Anschluss für Ratsche, für Hämmern geeignete Oberfläche). Dabei ist es vorteilhaft als Verbindungsstruktur des Implantats durch die Implantatfunktion bereits gegebene Strukturen zu verwenden oder die Verbindungsstrukturen am Implantat derart einfach zu gestalten (z.B. als Nut, Kamm, Oberflächenrauheit), dass das Implantat trotzdem optimal für seine Implantatfunktion ausgebildet ist. Die Verbindungsstruktur des Zwischenstücks besteht beispielsweise aus einer Mehrzahl von Klemmelementen, die einen Implantatkopf umgreifen oder aus einer Mehrzahl von Spreizelementen, die in eine beispielsweise hinterschnittene Kavität des Implantats eingreifen, wobei die Klemm- oder Spreizelemente den Werkstoff mit Formgedächtnis aufweisen, also das Formgedächtnis-Element darstellen, oder ein die Klemm- oder Spreizelemente in einer klemmenden oder gespreizten Konfiguration haltendes Formgedächtnis-Element vorgesehen ist. Für eine vorgespannte Verbindung weisen die Klemm- oder Spreizelemente mindestens bereichsweise ein elastisch deformierbares Material auf.

In einem zweiten Aspekt der Erfindung ist das Formgedächtnis-Element Teil einer Verbindungsstruktur, die in einem distalen Bereich eines Implantationswerkzeugs angeordnet ist. Das Werkzeug ist beispielsweise eine Sonotrode, deren distales Ende die Verbindungsstruktur aufweist und die an einen Schwingungsantrieb anschliessbar ist. Die Verbindungsstruktur am Werkzeug ist dabei im wesentlichen gleich ausgestaltet, wie oben für die Verbindungsstruktur am Zwischenstück beschrieben ist.

In einem dritten Aspekt der Erfindung ist das Formgedächtnis-Element Teil der am Implantat angeordneten Verbindungsstruktur, das heisst, es bleibt mit dem Implantat im Körper. Nach der Implantation, also wenn das Werkzeug oder Zwischenstück vom Implantat getrennt ist, kann die Verbindungsstruktur des Implantates gegebenenfalls eine weitere Verbindungsfunktion zur Verbindung eines weiteren Elements mit dem Implantat übernehmen, wobei das weitere Element bezüglich Verbindungsstruktur im wesentlichen gleich ausgerüstet ist wie das Werkzeug oder Zwischenstück. Auf einem entsprechend ausgerüsteten Dentalimplantat oder Wurzelstift könnte beispielsweise ein Abutment, eine Krone, Brücke oder Zahnprothese befestigt werden. Für eine reversible Befestigung des weiteren Elements (Zahnprothese an Zahnstumpf, wie beispielsweise beschrieben in JP1209059) ist ein Werkstoff mit Formgedächnis vorteilhaft, der einen intrinsischen Zweiwegeffekt aufweist. Weitere ähnliche Anwendungsbeispiele sind ein Anker, an dem als weiteres Element ein auswechselbares Hilfsaggregat (z.B. Sensor oder Impulsgeber) oder Medikamentendepot befestigt wird, oder ein implantierter künstlicher Darmausgang, an dem als weiteres Element ein auswechselbarer Plastikbeutel befestigt wird. Dabei gilt auch für die Befestigung und gegebenenfalls das Lösen des weiteren Elements, dass dabei keine Kraft auf das Implantat wirkt, die Schmerzen verursachen könnte.

Zwischenstück oder Werkzeug gemäss dem ersten, zweiten oder dritten Aspekt der Erfindung können einen Kanal mit distaler Mündung aufweisen, durch den dem Implantat oder seiner Umgebung für die Formwandlung des Formgedächnis-Elements eine warme oder kalte Flüssigkeit zur Wärmeübertragung zugeführt wird.

Im folgenden werden beispielhafte Ausführungsformen der Erfindung anhand von Zeichnungen im Detail erläutert, wobei die Erfindung nicht auf diese Ausführungsformen beschränkt ist, und insbesondere auch Kombinationen verschiedener Ausführungsformen möglich sind. Die Figuren zeigen:
- **Fig. 1**: Schematische Darstellung einer Implantationsvorrichtung mit Werkzeug, Zwischenstück und an das Zwischenstück koppelbarem Implantat, wobei die Vorrichtung beispielsweise geeignet ist für eine Implantation mit Hilfe eines durch mechanische Vibration verflüssigbaren Materials und mechanischer Vibration.
- **Fig. 2a bis 2e**: **A**xiale Schnitte und ein beispielhafter Querschnitt durch eine Mehrzahl von beispielhaften Ausführungsformen der erfindungsgemässen Implantationsvorrichtung mit einem Zwischenstück und an das Zwischenstück gekoppeltem Implantat (z.B. Dentalimplantat), welche Implantationsvorrichtung insbesondere geeignet ist für eine Implantation mittels durch mechanische Vibration verflüssigbarem Material und mechanischer Vibration.
- **Fig. 3a und 3b**: Ein axialer Schnitt und ein Querschnitt durch eine weitere beispielhafte Ausführungsform der erfindungsgemässen Implantationsvorrichtung mit schraubenförmigem Implantat, das durch Einschrauben implantiert wird.
- **Fig. 4**: Axialer Schnitt durch eine weitere beispielhafte Ausführungsform der erfindungsgemässen Implantationsvorrichtung mit einem Zwischenstück, das weitere Funktionen hat.

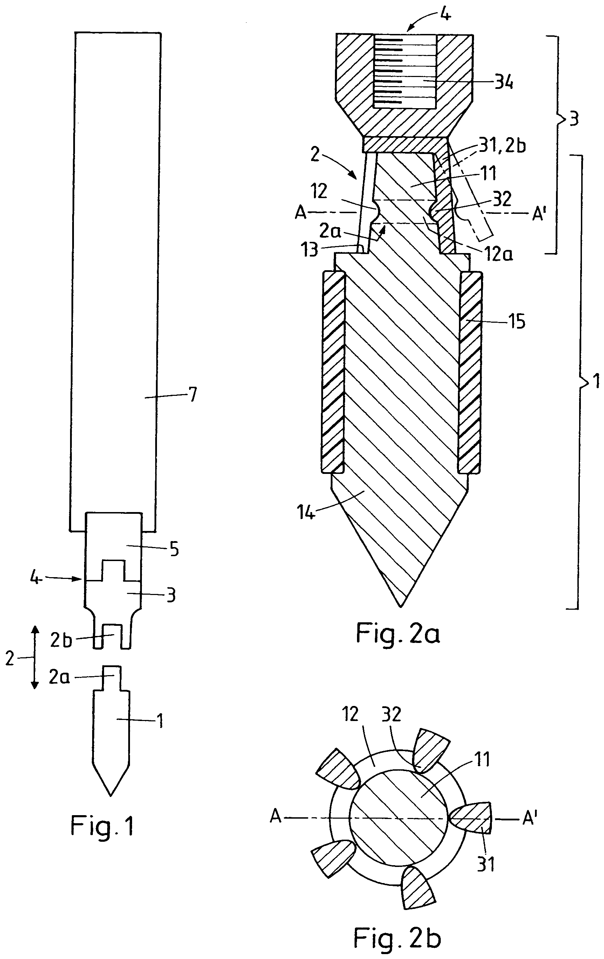

**Figur 1** zeigt eine schematische Darstellung einer Implantationsvorrichtung mit einer Verbindung 2 zwischen einer Verbindungsstruktur 2a an einem Implantat 1 und einer mit der Verbindungsstruktur 2a kooperierenden distalen Verbindungsstruktur 2b eines Zwischenstückes 3, wobei die eine oder andere Verbindungsstruktur 2a oder 2b ein in Figur 1 nicht dargestelltes Formgedächtnis-Element aufweist. Das Zwischenstück 3 ist beispielsweise mittels Schraubverbindung 4 an einem Werkzeug 5 befestigt. Das Werkzeug 5 kann ein- oder mehrteilig sein und ist beispielsweise eine Sonotrode, die für die Implantation an einen Ultraschallgenerator (Antrieb) angeschlossen wird, wobei das Gehäuse des Ultraschallgenerators als Handgriff 7 dient. In der in Fig. 1 dargestellten Implantationsvorrichtung kann das Zwischenstück auch fehlen, das heisst, das Implantat kann auch in derselben Weise wie an das Zwischenstück direkt an die Sonotrode gekoppelt sein.

**Figuren 2a bis 2e** zeigen axiale Schnitte beziehungsweise **Figur 2b** einen Querschnitt durch beispielhafte Ausführungsformen der erfindungsgemässen Implantationsvorrichtung mit Zwischenstück 3 in lösbarer Verbindung 2 mit einem Implantat 1, das beispielsweise ein Dentalimplantat ist und für eine Implantation mit mechanischen Schwingungen an der Oberfläche eines Implantatkörpers 14 ein durch die mechanischen Schwingungen verflüssigbares Material 15 aufweist. Zum Anschluss an ein Werkzeug oder für eine Kooperation damit weist das Zwischenstück 3 beispielsweise ein Gewinde 34 oder einen Innensechskant 35 auf.

Die distale Verbindungsstruktur 2b des Zwischenstücks weist Wandsegmente 31 eines Hohlkegelstumpfes auf, die einen konischen Implantatkopf 11 umgreifen und sich vorteilhafterweise auf einer Implantatschulter 13 abstützen und die den Werkstoff mit Formgedächtnis aufweisen.

In Figuren 2a und 2b sind die Wandsegmente 31 mit Ausformungen 32 ausgerüstet, welche in Ausnehmungen 12, beispielsweise in eine um den Implantatkopf 11 verlaufende Nut eingreifen und dadurch einen Formschluss gewährleisten. Für eine vorgespannte Verbindung sind die Wandsegmente elastisch und werden in der verbindenden Konfiguration gegen den Implantatkopf gepresst, wodurch zusätzlich ein Kraftschluss erzielt wird. Diese Ausführungsform gewährleistet eine starre, spielfreie Verbindung 2 und eignet sich zur im wesentlichen verlustfreien Übertragung der Stoss- und Zugbewegung von Ultraschallschwingungen. Die Verbindung eignet sich insbesondere für die Implantation von Dentalimplantaten, denn sie generiert im Betrieb wenig oder keine Sekundärschwingungen, die nicht nur einen Energieverlust darstellen, sondern im hörbaren Bereich liegen können und deshalb für den Patienten störend oder unangenehm sind.

In der Figur 2a sind die Wandsegmente 31 des Zwischenstücks in der verbindenden Konfiguration (z.B. Martensitstruktur des Werkstoffs mit Formgedächtnis) mit ausgezogenen Linien dargestellt. Eine Temperaturveränderung (Temperaturerhöhung), bewirkt einen Strukturübergang und eine Formänderung, durch die die Wandelemente sich aufweiten und den Formschluss auflösen (nichtverbindende Konfiguration rechts mit strich-punktierten Linien dargestellt).

Wenn die in der Figur 2a dargestellte Verbindung mit Spiel und ohne Klemmdruck ausgelegt ist, wird das Zwischenstück bei einer Implantation mit Ultraschall den Implantatkopf führen und auf das Implantat hämmern. Für eine solche Spielpassung ist der Implantatkopf 11 vorteilhafterweise zylinderförmig statt konisch und die Wandsegmente 31 bilden einen Hohlzylinder statt einen Hohlkegelstumpf. Ferner ist die Nut 12 grösser als die Ausformungen 32 der Wandsegmente 31.

In weiteren nicht in einer Figur dargestellten Ausführungsvarianten greifen den Wandsegmenten entsprechende Spreizelemente der Verbindungsstruktur des Zwischenstücks in eine Vertiefung im Implantat ein, wobei die Vertiefung für einen Formschluss hinterschnitten ist.

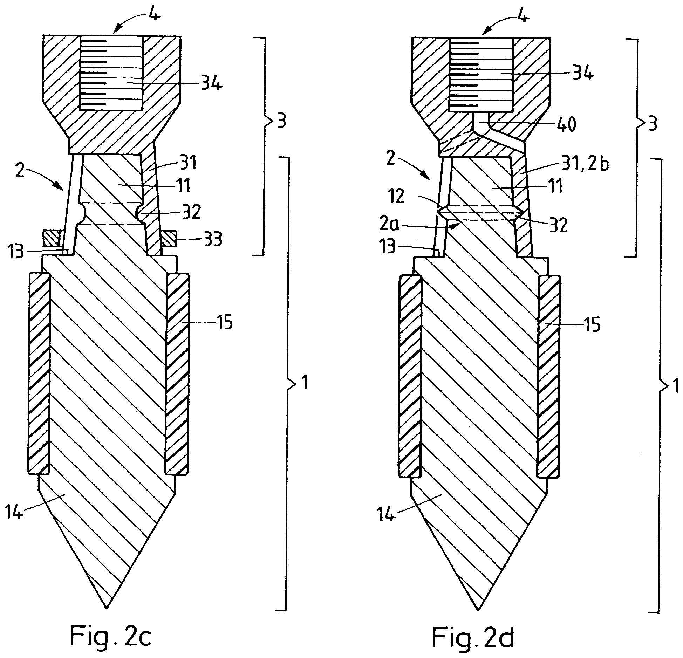

Gemäss **Figur 2c** gewährleisten die Wandsegmente 31 einen gleichen Formschluss wie gemäss Fig. 2a, weisen aber den Werkstoff mit Formgedächtnis nicht auf. Das Formgedächtnis-Element ist in dieser Ausführungsform ein aufgeschnittener Ring 33, der in der verbindenden Konfiguration einen kleinen Durchmesser hat und die Wandsegmente 32 gegen den Implantatkopf hält und der durch die Formveränderung grösser wird und dadurch ein Wegfedern der Wandelemente vom Implantatkopf bewirkt. Wenn der Ring 33 elastisch deformierbar ist, kann er die Wandelemente auch gegen den Implantatkopf vorspannen.

**Figur 2d** zeigt Wandsegmente 31 mit Ausnehmungen 32, die zusammen mit Ausformungen 12 (z.B. um den Implantatkopf verlaufende Rippe) des Implantatkopfes 11 einen Formschluss bilden. Ferner zeigt die Figur 2c einen Kanal 40, durch den eine Flüssigkeit zur Aufwärmung oder Kühlung des Formgedächnis-Elements zugeführt werden kann. Der dargestellte Kanal mündet im Bereich des Materials mit Formgedächtnis an die Oberfläche des Zwischenstücks, so dass die Flüssigkeit dessen Oberfläche überfliessen kann. Die Kanalmündung an der proximalen Seite des Zwischenstücks ist auf einen entsprechenden Kanal im Werkzeug ausgerichtet. Flüssigkeitszuführungen durch Sonotroden von im dentalen Bereich eingesetzten Ultraschallgeräten sind an sich bekannt. Es ist auch möglich, ein geschlossenes Kanalsystem für die wärmende oder kühlende Flüssigkeit vorzusehen.

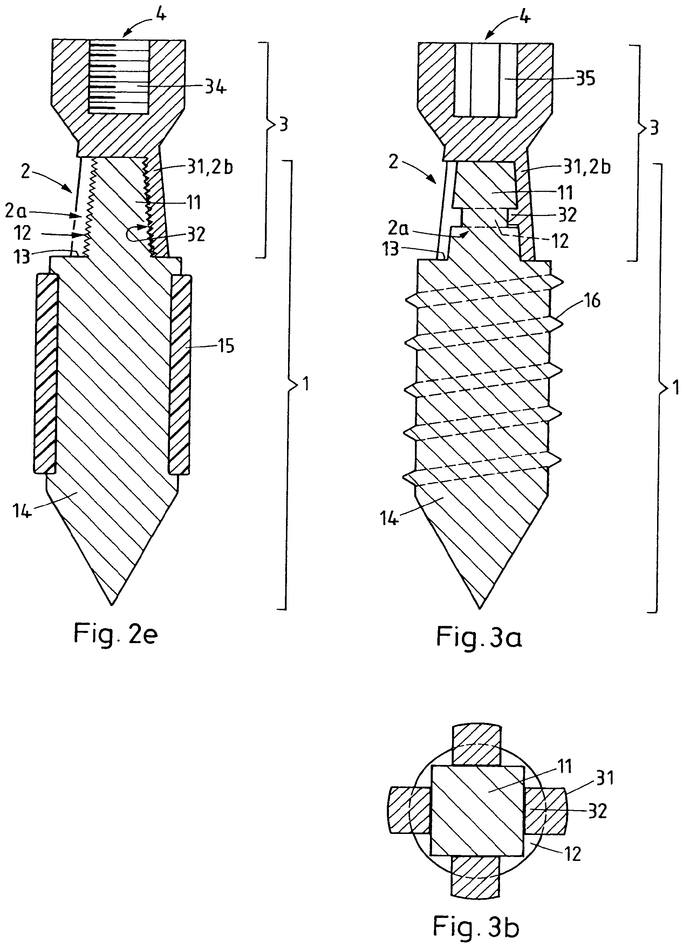

Gemäss **Figur 2e** weisen weder die Wandsegmente noch der Implantatkopf Ein- oder Ausformungen auf aber die Innenoberfläche der Wandsegmente und/oder die Oberfläche 12 des Implantatkopfes 11 weisen Mikrokerben oder eine rauhe Beschichtung 12 auf, sodass beim Aufeinanderpressen ein Mikroformschluss und gegebenenfalls Reibschluss entsteht. Die Oberflächenrauheit kann beispielsweise durch Beschichtung mit Hartstoffkörnern wie Diamant- oder Borcarbidkörnern erzeugt werden.

**Figuren 3a und 3b** zeigen einen Längs- und einen Querschnitt durch eine weitere beispielhafte Ausführungsform der erfindungsgemässen Implantationsvorrichtung, die wiederum ein Zwischenstück 3 in lösbarer Verbindung 2 mit einem Implantat 1 (Dentalimplantat) aufweist, wobei das Implantat 1 zum Einschrauben in Knochengewerbe mit einem Gewinde 14 ausgerüstet ist und das Zwischenstück proximal einen Innensechskant 35 aufweist, der ausgelegt ist, um für die Implantation z.B. mit einer Ratsche zu kooperieren. Die Verbindung 2 zwischen dem Zwischenstück 3 und dem Implantat 1 weist wiederum Wandsegmente 31 mit Anformungen 32 und am Implantatkopf Ausnehmungen 12 auf, wobei der Implantatkopf im Bereich der Ausnehmungen 12 eine vierkantige Form hat und dadurch die Wandelemente das Drehmoment im wesentlichen ohne Schlupf auf das Implantat übertragen können.

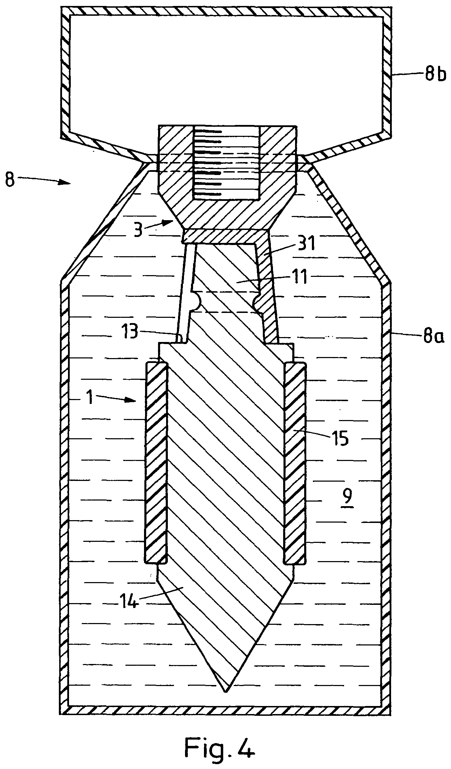

**Figur 4** zeigt eine weitere beispielhafte Ausführungsform der erfindungsgemässen Implantationsvorrichtung mit Zwischenstück 3 und Implantat 1. Das Zwischenstück 3 hat in dieser Ausführungsform neben der Adapter-Funktion zwischen Werkzeug und Implantat auch eine Funktion, die mit der Verpackung zusammenhängt. Die Verpackung 8 weist beispielsweise zwei Behälter 8a und 8b oder Beutel auf, deren Öffnungsrand das Zwischenstück derart umschliessen, dass das Implantat und das Zwischenstück völlig verpackt sind. Das Zwischenstück weist für den Anschluss der Behälteröffnungen beispielsweise zwei Nuten (nicht dargestellt) auf. Der Behälter 8a umschliesst das Implantat 1 und mindestens die distale Verbindungsstruktur 2a des Zwischenstücks. Der Behälter 8b umschliesst den Rest des Zwischenstücks, könnte aber auch fehlen.

Die Implantationsvorrichtung wird beispielsweise herstellerseitig steril verpackt und in verpacktem Zustand in den Handel gebracht und gelagert, wobei der Implantationsbehälter 8a gegebenenfallls eine für die Lagerung des Implantats vorteilhafte Flüssigkeit 9 enthalten kann. Als vorbereitender Schritt der Implantation wird der Behälter 8b entfernt und das Zwischenstück an ein Werkzeug angeschlossen. Für die Implantation selbst wird dann auch der Implantatbehälter 8a entfernt. Das Implantat muss dabei für keine der Manipulationen berührt werden.

Beispielhafte Anwendungsformen der Implantationsvorrichtung, des Implantes und des Werkzeugs gemäss einigen Ausführungsformen der Erfindung werden im Folgenden weiter erläutert mit einer Reihe von Sätzen:
1. Implantationsverfahren, in dem ein Implantat (1) in oder an einem menschlichen oder tierischen Körper positioniert und gegebenenfalls befestigt wird und in dem eine Implantationsvorrichtung nach Satz 1 verwendet wird, mit den folgenden Schritte:
   Zurverfügungstellen der Implantationsvorrichtung, in der das Implantat (1) an das Werkzeug (5) oder Zwischenstück (3) gekoppelt ist,
   Implantieren des Implantates (1) mit Hilfe des Werkzeuges (5) oder Zwischenstücks (3),
   Entkoppeln und Trennen des Werkzeugs (5) oder Zwischenstücks (3) vom Implantat, wozu das Formgedächtnis-Element und/oder dessen Umgebung auf eine physiologisch akzeptable Temperatur erwärmt oder abgekühlt, wird, um die Verbindungsstrukturen (2a, 2b) in ihre nicht-verbindende Konfiguration zu überführen.
2. Implantationsverfahren nach Satz 1, wobei das Formgedächtnis-Element erwärmt oder abgekühlt wird durch Zuführung einer Flüssigkeit mit einer entsprechenden Temperatur.
3. Implantationsverfahren nach Satz 1 oder 2, wobei der Schritt des Zurverfügungstellens eine Kopplung des Implantats (1) an das Werkzeug (5) oder Zwischenstück (3) beinhaltet.
4. Implantationsverfahren nach einem der Sätze 1 bis 3, wobei der Schritt des Zurverfügungstellens ein Anschliessen des Werkzeuges (5) an einen Antrieb beinhaltet.
5. Implantationsverfahren nach einem der Sätze 1 bis 4, wobei das Formgedächtnis-Element am Implantat (1) angeordnet ist und dass nach dem Schritt des Entkoppelns und Trennens in einem weiteren Schritt ein weiteres mit einer Verbindungsstruktur ausgerüstetes Element am implantierten Implantat befestigt wird.

## Patentansprüche

1. Implantationsvorrichtung für den Dentalbereich mit einem Implantationswerkzeug (5) oder einem an einem Implantationswerkzeug befestigbaren oder damit kooperierend ausgestalteten Zwischenstücks (3) und einem Implantat (1), **dadurch gekennzeichnet, dass** das Implantat (1) mit Hilfe von am Werkzeug (5) oder am Zwischenstück (3) und am Implantat (1) angeordneten Verbindungsstrukturen (2a, 2b) für die Implantation an das Werkzeug (5) oder an das Zwischenstück (3) gekoppelt oder koppelbar ist und nach der Implantation vom Werkzeug oder Zwischenstück getrennt ist, wobei mindestens eine der Verbindungsstrukturen (2a, 2b) ein Formgedächtnis-Element mit einem Werkstoff mit Formgedächtnis) aufweist, durch das die mindestens eine Verbindungsstruktur (2a, 2b) bei einem Übergang von einer Umgebungs- oder Körpertemperatur zu einer physiologisch akzeptablen Temperatur, die tiefer oder höher ist als die Umgebungs- oder die Körpertemperatur von einer verbindenden in eine nicht-verbindende Konfiguration überführbar ist, wobei die physiologisch akzeptable Temperatur, die höher ist als die Umgebungs- oder Körpertemperatur, zwischen 35 und 50°C liegt oder wobei die physiologisch akzeptable Temperatur, die tiefer ist als die Umgebungs- oder Körpertemperatur, zwischen 5 und -10°C liegt.

2. Implantationsvorrichtung nach Anspruch 1 **dadurch gekennzeichnet, dass** die zwei Verbindungsstrukturen (2a, 2b) für eine starre, vorgespannte form- und/oder kraftschlüssige Verbindung ausgelegt sind.

3. Implantationsvorrichtung nach Anspruch 1, **dadurch gekennzeichnet, dass** die Verbindungsstrukturen (2a, 2b) in der verbindenden Konfiguration ineinander greifen und/oder gegeneinander gepresst sind.

4. Implantationsvorrichtung nach Anspruch 1, **dadurch gekennzeichnet, dass** die Verbindungsstrukturen (2a, 2b) für eine formschlüssige Verbindung mit Spiel ausgelegt sind.

5. Implantationsvorrichtung nach einem der Ansprüche 2 oder 3, **dadurch gekennzeichnet, dass** die eine der Verbindungsstrukturen (2b) Wandsegmente (31) eines Hohlkörpers aufweist, die in der verbindenden Konfiguration an den anderen Verbindungsstrukturen (2a) anliegen oder gegen diese gepresst sind.

6. Implantationsvorrichtung nach Anspruch 5, **dadurch gekennzeichnet, dass** die Wandsegmente (31) Ausformungen (32) und die anderen Verbindungsstrukturen (2a) an die Ausformungen (32) angepasste Ausnehmungen (12) aufweisen, dass die Wandsegmente (31) Ausnehmungen und die anderen Verbindungsstrukturen (2a) an die Ausnehmungen angepasste Ausformungen aufweisen, oder dass Innenseiten der Wandsegmente (31) und/oder Oberflächenbereiche der anderen Verbindungsstrukturen (2a) Mikrostrukturen oder eine Rauheit aufweisen.

7. Implantationsvorrichtung nach Anspruch 5 oder 6, **dadurch gekennzeichnet, dass** die Wandsegmente (31) mindestens teilweise aus dem Werkstoff mit Formgedächtnis bestehen oder dass die Verbindungsstruktur mit den Wandsegmenten ferner einen Ring (33) aufweist, der die Wandsegmente (31) umgreifend angeordnet ist und der mindestens teilweise aus dem Werkstoff mit Formgedächtnis besteht.

8. Implantationsvorrichtung nach einem der Ansprüche 1 bis 7, **dadurch gekennzeichnet, dass** das Implantat (1) für eine Implantation mittels durch mechanische Vibration verflüssigbarem Material und mechanischer Vibration geeignet ist und das Werkzeug (5) eine Sonotrode ist oder das Zwischenstück (3) für eine Kopplung an eine Sonotrode ausgerüstet ist.

9. Implantationsvorrichtung nach einem der Ansprüche 1 bis 7, **dadurch gekennzeichnet, dass** das Zwischenstück (3) für eine Übertragung eines Drehmoments auf das Implantat (1) ausgerüstet ist.

10. Implantationsvorrichtung nach einem der Ansprüche 1 bis 9, **dadurch gekenntzeichnet** dass das Implantat (1) ein Dentalimplantat, ein Wurzelstift, ein Abutment, eine Zahnkrone oder eine Zahnfüllung ist.

11. Implantationsvorrichtung nach einem der Ansprüche 1 bis 10, **dadurch gekennzeichnet, dass** das Implantat (1) an das Werkzeug (5) oder Zwischenstück (3) gekoppelt ist und dass das Implantat (1) in einem Behälter (8a) oder Beutel angeordnet ist, wobei ein Rand einer Behälter- oder Beutelöffnung rund um das Werkzeug (5) oder das Zwischenstück (3) eng anliegend angeordnet ist.

12. Implantationsvorrichlung nach Anspruch 11, **dadurch gekennzeichnet, dass** ein proximaler Teil des Werkzeugs (5) oder Zwischenstücks (3) in einem weiteren Behälter (8b) oder Beutel angeordnet ist, wobei ein Rand einer Öffnung des weiteren Behälters oder Beutels rund um das Werkzeug (5) oder das Zwischenstück (3) eng anliegend angeordnet ist.

13. Implantat für den Dentalbereich, insbesondere ein Dentalimplantat, ein Wurzelstift, ein Abutment, eine Zahnkrone oder eine Zahnfüllung, **dadurch gekennzeichnet, dass** am Implantat (1)eine Verbindungsstruktur, die ein Formgedächtnis-Element aufweist, angeordnet ist für eine Verbindung an ein Werkzeug oder ein Zwischenstück einer Implantationsvorrichtung nach einem der Ansprüche 1 bis 7, wobei das Formgedächtnis-Element ein Werkstoff mit Formgedächtnis aufweist, durch das die Verbindungsstruktur bei einem Übergang von einer Umgebungs- oder Körpertemperatur zu einer physiologisch akzeptablen Temperatur, die tiefer oder höher ist als die Umgebungsoder die Körpertemperatur von einer verbindenden in eine nichtverbindende Konfiguration überführbar ist, wobei die physiologisch akzeptable Temperatur, die höher ist als die Umgebungs- oder Körpertemperatur, zwischen 35 und 50°C liegt oder wobei die physiologisch akzeptable Temperatur, die tiefer ist als die Umgebungs- oder Körpertemperatur, zwischen 5 und -10°C liegt.

14. Werkzeug (5) oder Zwischenstück (3) geeignet für eine Implantationsvorrichtung für den Dentalbereich nach einem der Ansprüche bis 7, **dadurch gekennzeichnet, dass** in einem distalen Bereich des Werkzeugs (5) oder Zwischenstücks (3) eine Verbindungsstruktur angeordnet ist, die ein Formgedächtnis-Element aufweiset zur Kopplung des Werkzeugs oder Zwischenstücks an ein Implantat für den Dentalbereich, insbesondere an ein Dentalimplantat, einen WurzeIstift, ein Abutment, eine Zahnkrone oder eine Zahnfüllung, wobei das Formgedächtnis-Element ein Werkstoff mit Formgedächtnis aufweist, durch das die Verbindungsstruktur bei einem Übergang von einer Umgebungs- oder Körpertemperatur zu einer physiologisch akzeptablen Temperatur, die tiefer oder höher ist als die Umgebungs- oder die Körpertemperatur von einer verbindenden in eine nichtverbindende Konfiguration überführbar ist, wobei die physiologisch akzeptable Temperatur, die höher ist als die Umgebungsoder Körpertemperatur, zwischen 35 und 50°C liegt oder wobei die physiologisch akzeptable Temperatur, die tiefer ist als die Umgebungs- oder Körpertemperatur, zwischen 5 und -10°C liegt.

## Claims

1. An implantation device for the dental field, with an implantation tool (5) or with an intermediate piece (3) which is fastenable to an implantation tool or cooperates therewith, and with an implant (1), **characterised in that** for the implantation, the implant (1) is coupled or can be coupled onto the tool (5) or onto the intermediate piece (3) with the help of connecting structures (2a, 2b) which are arranged on the tool (5) or on the intermediate piece (3) and on the implant (1), and after the implantation is separated from the tool or intermediate piece, wherein at least one of the connecting structures (2a, 2b) is a shape memory element with a material having a shape memory, by way of which the at least one connecting structure (2a, 2b), given a transition from a surrounding temperature or body temperature to a physiologically acceptable temperature which is lower or higher than the surrounding temperature or the body temperature, can be brought from a connecting into a non-connecting configuration, wherein the physiologically acceptable temperature which is higher than the surrounding temperature or body temperature lies between 35 and 50° or wherein the physiologically acceptable temperature which is lower than the surrounding temperature or body temperature lies between 5 and -10°C.

2. An implantation device according to claim 1, **characterised in that** the two connecting structures (2a, 2b) are designed for a rigid, prestressed positive and/or non-positive connection.

3. An implantation device according to claim 1, **characterised in that** in the connecting configuration, the connecting structures (2a, 2b) engage into one another or are pressed against one another.

4. An implantation device according to claim 1, **characterised in that** the connecting structures (2a, 2b) are designed for a positive connection with play.

5. An implantation device according to one of the claims 2 or 3, **characterised in that** the one of the connecting structures (2b) comprises wall segments (31) of a hollow body which in the connecting configuration bear on the other connecting structures (2a) or are pressed against these.

6. An implantation device according to claim 5, **characterised in that** the wall segments (31) comprises protuberances (32) and the other connecting structures (2a) comprise recesses (12) which are matched to the protuberances (32), that the wall segments (31) comprise recesses and the other connecting structures (2a) comprise protuberances which are matched to the recesses, or that inner sides of the wall segments (31) and/or surface regions of the other connecting structures (2a) comprise microstructures or a roughness.

7. An implantation device according to claim 5 or 6, **characterised in that** the wall segments (31) at least partly consist of the material with the shape memory or that the connecting structure with the wall segments moreover comprises a ring (33) which is arranged in a manner embracing the wall segments (31) and which at least partly consists of the material with shape memory.

8. An implantation device according to one of the claims 1 to 7, **characterised in that** the implant (1) is suitable for an implantation by way of material which is liquifiable by mechanical vibration and by way of mechanical vibration and the tool (5) is a sonotrode or the intermediate piece (3) is designed for coupling onto a sonotrode.

9. An implantation device according to one of the claims 1 to 7, **characterised in that** the intermediate piece (3) is designed for a transmission of a torque onto the implant (1).

10. An implantation device according to one of the claims 1 to 9, **characterised in that** the implant (1) is a dental implant, a root pin, an abutment, a dental crown or a dental filling.

11. An implantation device according to one of the claims 1 to 10, **characterised in that** the implant (1) is coupled onto the tool (5) or intermediate piece (5) and that the implant (1) is arranged in a container (8a) or pouch, wherein an edge of a container opening or pouch opening is arranged bearing snugly around the tool (5) or the intermediate piece (3).

12. An implantation device according to claim 11, **characterised in that** a proximal part of the tool (5) or of the intermediate piece (3) is arranged in a further container (8b) or pouch, wherein an edge of an opening of the further container or of the pouch is arranged bearing snugly around the tool (5) or the intermediate piece (3).

13. An implant for a dental field, in particular a dental implant, a root pin, an abutment, a dental crown or a dental filling, **characterised in that** a connecting structure which comprises a shape memory element is arranged on the implant (1), for a connection onto a tool or onto an intermediate piece of an implantation device according to one of the claims 1 to 7, wherein the shape memory element comprises a material with a shape memory, by way of which the connecting structure, given a transition from a surrounding temperature or body temperature to a physiologically acceptable temperature which is lower or higher than the surrounding temperature or the body temperature, can be brought from a connecting into a non-connecting configuration, wherein the physiologically acceptable temperature which is higher than the surrounding temperature or body temperature lies between 35 and 50° or wherein the physiologically acceptable temperature which is lower than the surrounding temperature or body temperature lies between 5 and -10°C.

14. A tool (5) or an intermediate piece (3), suitable for an implantation device for the dental field according to one of the claims 1 to 7, **characterised in that** a connecting structure is arranged in a distal region of the tool (5) or intermediate piece (3), said connecting structure comprising a shape memory element for coupling the tool or the intermediate piece onto an implant for the dental field, in particular onto a dental implant, a root pin, an abutment, a dental crown or a dental filling, wherein the shape memory element comprises a material with a shape memory, by way of which the connecting structure, given a transition from a surrounding temperature or body temperature to a physiologically acceptable temperature which is lower or higher than the surrounding temperature or the body temperature, can be brought from a connecting into a non-connecting configuration, wherein the physiologically acceptable temperature which is higher than the surrounding temperature or body temperature lies between 35 and 50° or wherein the physiologically acceptable temperature which is lower than the surrounding temperature or body temperature lies between 5 and -10°C.

## Revendications

1. Dispositif d'implantation pour le secteur dentaire, comprenant un outil d'implantation (5) ou une pièce intermédiaire (3) configurée pour pouvoir être fixée à un outil d'implantation ou coopérer avec celui-ci et un implant (1), **caractérisé en ce que** l'implant (1) est accouplé ou peut être accouplé à l'outil (5) ou à la pièce intermédiaire (3) pour l'implantation à l'aide de structures de liaison (2a, 2b) disposées sur l'outil (5) ou sur la pièce intermédiaire (3) et sur l'implant (1), et il est séparé de l'outil ou de la pièce intermédiaire après l'implantation, au moins l'une des structures de liaison (2a, 2b) possédant un élément à mémoire de forme avec un matériau à mémoire de forme par le biais duquel l'au moins une structure de liaison (2a, 2b) peut être amenée d'une configuration de connexion à une configuration de non connexion lors d'une transition d'une température ambiante ou corporelle à une température physiologiquement acceptable, laquelle est inférieure ou supérieure à la température ambiante ou corporelle, la température physiologiquement acceptable qui est supérieure à la température ambiante ou corporelle étant comprise entre 35 °C et 50 °C ou la température physiologiquement acceptable qui est inférieure à la température ambiante ou corporelle étant comprise entre 5 °C et -10 °C.

2. Dispositif d'implantation selon la revendication 1, **caractérisé en ce que** les deux structures de liaison (2a, 2b) sont conçues pour une liaison à complémentarité de formes et/ou par force précontrainte rigide.

3. Dispositif d'implantation selon la revendication 1, **caractérisé en ce que** dans la configuration de connexion, les structures de liaison (2a, 2b) viennent en prise l'une dans l'autre et/ou sont pressées l'une contre l'autre.

4. Dispositif d'implantation selon la revendication 1, **caractérisé en ce que** les structures de liaison (2a, 2b) sont conçues pour une liaison par complémentarité de formes avec jeu.

5. Dispositif d'implantation selon l'une des revendications 2 ou 3, **caractérisé en ce que** l'une des structures de liaison (2b) possède des segments de paroi (31) d'un corps creux qui, dans la configuration de connexion, reposant contre l'autre des structures de liaison (2a) ou sont pressés contre celle-ci.

6. Dispositif d'implantation selon la revendication 5, **caractérisé en ce que** les segments de paroi (31) possèdent des façonnages (32) et les autres structures de liaison (2a) des cavités (12) adaptées aux façonnages (12), **en ce que** les segments de paroi (31) possèdent des cavités et les autres structures de liaison (2a) des façonnages adaptés aux cavités, ou **en ce que** les côtés intérieurs des segments de paroi (31) et/ou des zones de paroi des autres structures de liaison (2a) possèdent des microstructures ou une rugosité.

7. Dispositif d'implantation selon la revendication 5 ou 6, **caractérisé en ce que** les segments de paroi (31) sont constitués au moins en partie du matériau à mémoire de forme ou **en ce que** la structure de liaison pourvue des segments de paroi possède en outre un anneau (33) qui est disposé de manière à envelopper les segments de paroi (31) et qui est constitué au moins en partie du matériau à mémoire de forme.

8. Dispositif d'implantation selon l'une des revendications 1 à 7, **caractérisé en ce que** l'implant (1) est conçu pour une implantation au moyen d'un matériau liquéfiable par vibration mécanique et de vibrations mécaniques et l'outil (5) est une sonotrode ou la pièce intermédiaire (3) est équipée pour un couplage à une sonotrode.

9. Dispositif d'implantation selon l'une des revendications 1 à 7, **caractérisé en ce que** la pièce intermédiaire (3) est équipée pour une transmission d'un couple à l'implant (1).

10. Dispositif d'implantation selon l'une des revendications 1 à 9, **caractérisé en ce que** l'implant (1) est un implant dentaire, un pivot radiculaire, un pilier, une couronne dentaire ou une obturation dentaire.

11. Dispositif d'implantation selon l'une des revendications 1 à 10, **caractérisé en ce que** l'implant (1) est couplé à l'outil (5) ou à la pièce intermédiaire (3) et **en ce que** l'implant (1) est disposé dans un récipient (8a) ou un sachet, un bord d'une ouverture de récipient ou de sachet étant disposé étroitement tout autour de l'outil (5) ou de la pièce intermédiaire (3).

12. Dispositif d'implantation selon la revendication 11, **caractérisé en ce qu'**une partie proximale de l'outil (5) ou de la pièce intermédiaire (3) est disposée dans un récipient (8b) ou un sachet supplémentaire, un bord d'une ouverture du récipient ou du sachet supplémentaire étant disposé étroitement tout autour de l'outil (5) ou de la pièce intermédiaire (3).

13. Implant pour le secteur dentaire, notamment un implant dentaire, un pivot radiculaire, un pilier, une couronne dentaire ou une obturation dentaire, **caractérisé en ce qu'**une structure de liaison, laquelle possède un élément à mémoire de forme, est disposée sur l'implant (1) pour une liaison à un outil ou une pièce intermédiaire d'un dispositif d'implantation selon l'une des revendications 1 à 7, l'élément à mémoire possédant un matériau à mémoire de forme par le biais duquel la structure de liaison peut être amenée d'une configuration de connexion à une configuration de non connexion lors d'une transition d'une température ambiante ou corporelle à une température physiologiquement acceptable, laquelle est inférieure ou supérieure à la température ambiante ou corporelle, la température physiologiquement acceptable qui est supérieure à la température ambiante ou corporelle étant comprise entre 35 °C et 50 °C ou la température physiologiquement acceptable qui est inférieure à la température ambiante ou corporelle étant comprise entre 5 °C et -10 °C.

14. Outil (5) ou pièce intermédiaire (3) conçu pour un dispositif d'implantation pour le secteur dentaire selon l'une des revendications 1 à 7, **caractérisé en ce qu'**une structure de liaison est disposée dans une zone distance de l'outil de la pièce intermédiaire (3) pour le couplage de l'outil ou de la pièce intermédiaire à un implant pour le secteur dentaire, notamment à un implant dentaire, un pivot radiculaire, un pilier, une couronne dentaire ou une obturation dentaire, l'élément à mémoire possédant un matériau à mémoire de forme par le biais duquel la structure de liaison peut être amenée d'une configuration de connexion à une configuration de non connexion lors d'une transition d'une température ambiante ou corporelle à une température physiologiquement acceptable, laquelle est inférieure ou supérieure à la température ambiante ou corporelle, la température physiologiquement acceptable qui est supérieure à la température ambiante ou corporelle étant comprise entre 35 °C et 50 °C ou la température physiologiquement acceptable qui est inférieure à la température ambiante ou corporelle étant comprise entre 5 °C et -10 °C.
